# EUROPEAN PATENT SPECIFICATION

(11) **EP 3 556 318 B1**
(45) Date of publication and mention of the grant of the patent: **01.09.2021**
(21) Application number: 18168524.9
(22) Date of filing: 20.04.2018
(51) Int. Cl.: A61B 90/30

(54) **OPERATING LIGHT**
OPERATIONSLEUCHTE
ÉCLAIRAGE DE SALLES OPÉRATOIRES

(43) Date of publication of application: 23.10.2019
(73) Proprietor: TRUMPF Medizin Systeme GmbH + Co. KG, 07318 Saalfeld (DE)
(72) Inventor: MEINDL, Susanne, 80339 München (DE)
(74) Representative: Prüfer & Partner mbB Patentanwälte · Rechtsanwälte

(56) References cited:
- WO-A1-2014/054772
- US-A1- 2010 081 887
- US-A1- 2011 009 694
- US-A1- 2015 351 637
- US-A1- 2017 318 644

## Description

The invention relates to an operating light, in particular, to an operating light that emits heat radiation to a surgical site.

A temperature rising in the surgical site is an everyday challenge for surgeons and nurses since tissues dry out or get damaged or burned if there is too much heat applied. However, except from visible light, operating lights can also emit heat radiation.

In particular, during the course of a long-term surgery, after a certain time, the surgeon might require more light due to fatigue of his eyes which brings the problem that tissue illuminated by the operating light which has already been irradiated for a long term is dried out and burned. However, since the attention of the surgeon and of operation personnel is focused on performing the surgery, there is the risk that the tissue can be unnoticeably dried out or burned.

In document US 2017/318644 A1, an operating light having a light head including several illuminants and a temperature sensor for detecting a temperature in a surgical field in order to avoid desiccation of a wound is disclosed.

Therefore, the object underlying the invention is to signalize the risk of damaging tissue illuminated by the operating light to the surgeon or other operating personnel.

The object is achieved by an operating light according to claim 1 and a method for detecting a temperature in a surgical site according to claim 11. Advantageous further developments are subject-manner of the dependent claims.

According to an aspect of the invention, an operating light comprises a light head including at least one illuminant for illuminating a surgical site and a temperature sensor attached to a component of the operating light for detecting a temperature in the surgical site in a touchless manner.

When the operating light comprises the temperature sensor, it is very easy to determine a risk of damaging tissue irradiated by the operating light. Therefore, there is less risk of tissue damage due to drying out or burning, and, thus, a better treatment is enabled. The heating of the tissue has a direct correlation with the use of the operating light, and, therefore, no additional structural components and additional operation for detecting the temperature in the surgical site are necessary.

In an advantageous implementation, the operating light comprises a first mechanical and electrical interface, the temperature sensor is a component of a temperature sensor assembly, and the temperature sensor assembly comprises a second mechanical and electrical interface configured to be coupled to the first mechanical and electrical interface.

By providing the first mechanical and electrical interface and the second mechanical and electrical interface, it is possible to easily attach or detach the temperature sensor as a component of the temperature sensor assembly to the operating lamp or from the lamp, wherein the mechanical and electrical connections and disconnections can simultaneously be performed.

In a further advantageous implementation, the first mechanical and electrical interface is arranged at the center of the light head on the side of the light emission surface of the light head.

Due to the arrangement of the temperature sensor at the center of the light head on the side of the light emission surface of the light head, the detection spot of the temperature sensor can be easily directed to the center of a light field generated by the operating light where the irradiation power is usually the highest and, therefore, the risk of drying or burning the tissue is the highest. Moreover, the temperature sensor can automatically be aligned with the light emitted by the operating light without the need of any further alignment operation. Therefore, if the light head is adjusted for adjusting a position of the light field, a detection spot of the temperature sensor is automatically aligned

In a further advantageous implementation, the first mechanical and electrical interface is configured to attach and to electrically connect a camera to the operating light.

When the first mechanical and electrical interface is configured in such a manner, the first mechanical and electrical interface adapted to attach and connect the camera to the operating light can also be used for attaching and electrically connecting the temperature sensor to the operating light. There is no need to provide a further interface if the temperature sensor is used instead of the camera.

In a further advantageous implementation, the temperature sensor is an infrared temperature sensor.

By using an infrared temperature sensor, a sensor being easy to operate and quite inexpensive can be used for detecting the temperature in the operating site.

In a further advantages implementation, the temperature sensor is a thermal imaging camera.

By using the thermal imaging camera as the temperature sensor, the heat distribution across the light field can be detected. Therefore, an area of the operating site illuminated by the light field, wherein the area, for example, is located in an arbitrary region of the light field, can be identified as an area having the highest temperature e.g. due to the kind of the tissue and, therefore, the highest risk of drying out and burning the tissue.

In a further advantageous implementation, the operating light is configured to verify whether the detected temperature is within a predefined temperature range.

By verifying whether the detected temperature is within the predefined temperature range, it is easy to recognize if the temperature sensor detects the temperature correctly within the operating site, or outside the operating site, and, furthermore, the correct function of the temperature sensor can be estimated.

According to the invention the operating light is configured to perform several successive temperature measurements and to determine a temperature difference between a previous temperature measurement and one of subsequent temperature measurements.

By a determination of the temperature difference between the temperature of the previous temperature measurement and the temperature of one of the subsequent temperature measurements, a temperature profile can be determined. Therefore, it is possible, starting from e.g. an initial temperature, to determine necessary actions due to the temperature profile.

Further according to the invention, the operating light is configured to perform at least one of a decreasing of light intensity of the operating light and of a signaling upon reaching or exceeding the predefined difference between the previous measurement and one of the subsequent temperature measurements.

When the operating light signals a high temperature or decreases its light intensity upon reaching or exceeding the predefined difference between the previous measurement and one of the subsequent temperature measurements, the operation personal can automatically be informed about an excessive temperature in the operating site or the light intensity of the operating lamp can automatically be decreased for reducing the thermal burden of the irradiated tissue.

In a further advantageous implementation, the operating light is provided with a motion sensor, and the operating light is configured to initiate a temperature measurement after repositioning of the light head.

By repositioning the light head, i.e. completing a motion of the light head, a modified area of the surgical site can be illuminated so that a measurement of the temperature of the modified area is relevant for determining the thermal burden of the tissue in order to avoid dry out and damage of the tissue.

According to a further aspect of the invention, a method for detecting a temperature in the surgical site by an operating light is provided. The method comprises the steps: performing the previous temperature measurement and the subsequent temperature measurements; determining a temperature difference between the temperature detected by the previous temperature measurement and a temperature detected by the subsequent temperature measurements; and performing at least one of the decreasing of light intensity of the operating light and of the signaling when the determined temperature difference reaches or exceeds the predefined temperature difference or a maximum predefined temperature.

By performing this method, it is very easy to determine and to avoid the risk of damaging the tissue irradiated by the operating light.

In an advantageous implementation of the method, it comprises the steps: verifying whether a temperature respectively detected in the previous measurement and in the subsequent measurements is in a predefined temperature range, and, when the detected temperature is out of the predefined temperature range, initiating a signaling of this situation.

By these steps, it is easy to recognize if the temperature sensor detects a temperature within or outside the operating site, and, furthermore, the correct function of the temperature sensor can be estimated.

In a further advantageous implementation of the method, the subsequent temperature measurements are automatically performed after the lapse of a constant predefined time.

By this method step, a representative temperature profile can be determined.

In a further implementation of the method, a time until the subsequent temperature measurement is performed is determined depending on the temperature difference between the previous temperature measurement and the subsequent temperature measurement.

By this method step, a faster and more exact determination of the temperature profile is possible when approaching the temperature to be signalized or at which the light intensity is to be decreased.

In a further implementation of the method, the first temperature measurement is initiated upon switching on the operating light.

When the first temperature measurement is initiated upon switching on the operating light, no further action is necessary for detecting an initial temperature in the operating site and there is no risk of forgetting performing the temperature measurement.

In a further implementation of the method, a subsequent temperature measurement is initiated as a previous temperature measurement after repositioning of the light head.

When, after repositioning of the light head, i.e. completing a motion of the light head, the subsequent temperature measurement is initiated as a previous temperature measurement, measurement of the temperature of a modified area of the surgical site can be the basis for determining the thermal burden of the tissue in order to avoid dry out and damage of the tissue.

The invention is now elucidated by means of an embodiment referring to the attached drawing.

In particular,
- Fig. 1: shows a principle illustration of an operating light 1 according to the invention; and
- Fig. 2: shows a graph for explaining the temperature behavior.

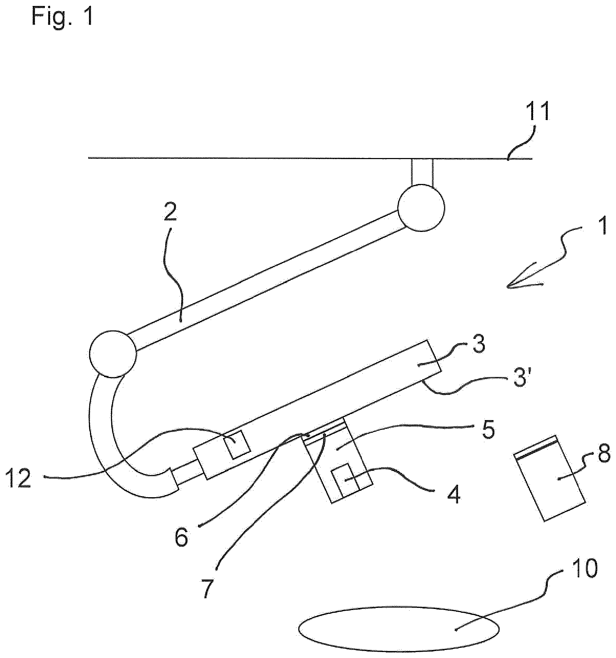

In Fig. 1, a principle illustration of an operating light 1 according to the invention is shown. The operating light 1 comprises a suspension system 2 for being installed to e.g. a ceiling 11 of an operating theater. Furthermore, the operating light 1 comprises a light head 3 including several illuminants for illuminating a surgical site 10. In this embodiment, the light head 3 comprises several illuminants, however, in an alternative embodiment, at least one illuminant is provided.

At the light head 3, a temperature sensor 4 for detecting a temperature in the surgical site 10 in a touchless manner is attached. Alternatively, the temperature sensor 4 can also be attached to the suspension system 2 as another member of the operating light 1. The temperature sensor 4 is a component of a temperature sensor assembly 5 which additionally comprises e.g. a housing and a fixing device. Alternatively, the temperature sensor 4 can also be solely attached to the operating light 1.

For attaching the temperature sensor 4, in particular the temperature sensor assembly 5, to the light head 2, the operating light 1 comprises a central coupling point as a first mechanical and electrical interface 6. The first mechanical and electrical interface 6 is arranged at the center of the light head 3 on a side of the light emissions surface 3' of the light head 3. The first mechanical and electrical interface 6 is also configured to attach and connect a camera 8 which is separately illustrated to the operating light 1. Therefore, the first mechanical and electrical interface 6 is a central coupling point of a camera prepared light head. Alternatively, the first mechanical and electrical interface 6 is not mandatorily configured to attach and to connect the camera 8 to the operating light 1 but it can be designed as a specific interface for the temperature sensor assembly 5, or, further alternatively, as an interface for the temperature sensor assembly and other potential attachments. Moreover, the first mechanical and electrical interface 6 can also be provided at another component of the operating light 1, e.g. at the suspension system 2.

The temperature sensor assembly 5 comprises a second mechanical and electrical interface 7 to be coupled to the first mechanical and electrical interface 6 of the light head 3. As the case may be, the temperature sensor assembly 5 can also be coupled to the first mechanical and electrical interface 6 at another component of the operating light 1.

In this embodiment, the temperature sensor 4 is an infrared temperature sensor. However, in alternative embodiments, the temperature sensor 4 can also be e.g. a thermal imaging camera or it can be a sensor based on another technique.

The operating light is provided with a motion sensor 12. In the present embodiment, the motion sensor 12 is arranged in the light head 3 for detecting a motion of the operating light 1, in particular of the light head 3. In alternative embodiments, the motion sensor 12 can also be arranged at another component of the operating light 1, e.g. at the suspension system 2.

The operating light 1 comprises a control such that the operating light 1 is configured to verify whether the detected temperature is within a predefined temperature range. The control comprises an algorithm determining whether the measured temperature is within a possible range of surgical tissue, so that measurements of a tabletop, a floor, etc. can be eliminated.

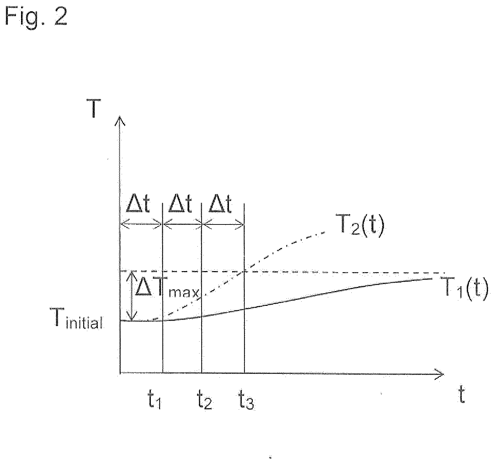

Fig. 2 shows a graph for explaining the temperature behavior.

An abscissa of the graph illustrates an elapsed time t. An ordinate indicates a temperature T in the surgical site.

At a start time ti, the temperature is an initial temperature Tᵢₙᵢₜᵢₐₗ measured by a first temperature measurement.

As shown in Fig. 2, the temperature T in the surgical site (10) is respectively measured after the lapse of a constant predefined time interval Δt, e.g., at a frequency of five minutes. Alternatively, a time interval until the subsequent temperature measurement is performed is determined depending on the temperature difference between the previous temperature measurement and the subsequent temperature measurement. Therefore, the time interval Δt can be increased if the temperature difference between two consecutive temperature measurements is small and the time interval Δt can be decreased if the temperature difference between two consecutive temperature measurements is large in order to enhance an accuracy and quickness of the determination of reaching and exceeding an allowed temperature.

A dotted horizontal line illustrates a value of a maximum permitted temperature difference ΔTₘₐₓ from the initial temperature without a risk of drying out and burning tissue irradiated by the operating light (1). The maximum permitted temperature difference ΔTₘₐₓ is a value which is empirically determined. Alternatively or additionally, a maximum permitted temperature Tₘₐₓ can also have an absolute value independent from an initial value.

A solid line illustrates a behavior of a first temperature T₁(t) which is a temperature that does not reach or exceed the temperature at the maximum permitted temperature difference ΔTₘₐₓ. Therefore, the operating light (1) can be operated without decreasing its light intensity, nevertheless, without creating the risk of drying out or burning the tissue.

A dash-and-dot line illustrates a behavior of a second temperature T₂(t) which is a temperature that exceeds the maximum permitted temperature difference ΔTₘₐₓ at a time t₃. Therefore, after the time t₃, there is the risk that the tissue dries out or is burned. The subsequent temperature measurement at t₃ would have the result that an alert signal is given or the light intensity of the operating light (1) is decreased, which is not shown in the graph.

The alert signal is a visual and an acoustic feedback, wherein, alternatively, also merely one of the visual and acoustic feedback is possible. The visual feedback is incorporated in the central handle behind a diffusing screen, however, can alternatively also be arranged at another suitable location, e.g. at a housing of the light head 3. The acoustic feedback is a short beep which is loud enough to be heard within a one meter distance of the light head 3 but which does not disturb. Alternatively, another sound is possible. The visual and/or acoustic feedback endure as long as the temperature T in the surgical site 10 does not decrease or until e.g. the surgeon resets the signal.

A factory-set predefined time interval Δt has to be evaluated if it does not fit to the present installation or to the present surgery.

In use, the temperature T in the surgical site 10 is detected by performing the previous temperature measurement and subsequent temperature measurements. Further, a temperature difference ΔT between a temperature detected by the previous temperature measurement the temperature detected by the subsequent temperature measurements is determined. Moreover, at least one of the decreasing of light intensity of the operating light 1 and of the signaling is performed when the temperature difference ΔT achieves or exceeds the predefined maximum temperature difference ΔTₘₐₓ. In the shown embodiment, the temperature of the previous temperature measurement at to is the initial temperature Tᵢₙᵢₜᵢₐₗ. Nevertheless, the temperature of the previous measurement can alternatively also be a temperature measured after a certain time interval after starting the measurements. Alternatively, the maximum permitted temperature Tₘₐₓ can also have an absolute value independent from an initial value.

As the case may be, it is verified whether the temperature respectively detected in the previous temperature measurement and the subsequent temperature measurements is in a predefined temperature range and, when the detected temperature is out of the predefined temperature range, a signaling of this situation as a fault condition is initiated.

Furthermore, the subsequent temperature measurements are automatically performed after a lapse of a constant predefined time Δt. Alternatively, a distance of time until the subsequent temperature measurement is performed is depending on the temperature difference between the previous temperature measurement and the subsequent temperature measurements. In further alternative embodiments, the subsequent temperature measurements are initiated by completing a movement of the light head, or by the lapse of the time and the movement of the light head. The first temperature measurement is initiated upon switching on the operating light. Nevertheless, the first temperature measurement can also be initiated by operating a sterile control option to set the reference starting temperature, e.g. by pressing a button of a central handle by the surgeon.

While the invention has been illustrated and described in detail in the drawing and in the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described therein.

The invention has been described in conjunction with various embodiments herein. However, other variations to the disclosed embodiments can be understood and effected by those skilled in the art in practicing the claimed invention, from a study of the drawing, the disclosure and dependent claims. In the claims, the word "comprising" does not exclude other elements or steps, and the indefinite article "a" or "an" does not exclude a plurality.

## Claims

1. Operating light (1) comprising
a light head (3) including at least one illuminant for illuminating a surgical site (10), and
a temperature sensor (4) attached to a component of the operating light (1) for detecting a temperature in the surgical site (10) in a touchless manner,
**characterized in that**
the operating light (1) is configured to perform several successive temperature measurements and to determine a temperature difference between a previous temperature measurement and one of subsequent temperature measurements,
wherein
the operating light (1) is configured to perform at least one of a decreasing of light intensity of the operating light (1) and a signaling upon reaching or exceeding a predefined difference between the previous temperature measurement and one of the subsequent temperature measurements.

2. Operating light (1) of claim 1, wherein
the operating light (1) comprises a first mechanical and electrical interface (6),
the temperature sensor (4) is a component of a temperature sensor assembly (5), and
the temperature sensor assembly (5) comprises a second mechanical and electrical interface (7) configured to be coupled to the first mechanical and electrical interface (6).

3. Operating light (1) of claim 2, wherein
the first mechanical and electrical interface (6) is arranged at the center of the light head (3) on a side of a light emission surface (3') of the light head (3).

4. Operating light (1) of claim 2 or 3 wherein
the first mechanical and electrical interface (6) is configured to attach and to electrically connect a camera to the operating light (1).

5. Operating light (1) of any preceding claim, wherein
the temperature sensor (4) is an infrared temperature sensor.

6. Operating light (1) of anyone of claims 1 to 4, wherein
the temperature sensor (4) is a thermal imaging camera.

7. Operating light (1) of any preceding claim, wherein
the operating light (1) is configured to verify if the detected temperature is within a predefined temperature range.

8. Operating light (1) of any preceding claim, wherein
the operating light (1) is provided with a motion sensor (12), and
the operating light (1) is configured to initiate a temperature measurement after repositioning of the light head (3).

9. Method for detecting a temperature in a surgical site (10) by an operating light (1) of any of claims 1 to 8 comprising the steps:
- performing a previous temperature measurement and subsequent temperature measurements;
- determining a temperature difference between a temperature detected by the previous temperature measurement and a temperature detected by the subsequent temperature measurements; and
- performing at least one of a decreasing of light intensity of the operating light (1) and of signaling when the determined temperature difference reaches or exceeds a predefined temperature difference or a maximum predefined temperature.

10. Method of claim 9 comprising the steps:
- verifying whether a temperature respectively detected in the previous temperature measurement and in the subsequent temperature measurements is in a predefined temperature range; and
- when the detected temperature is out of the predefined temperature range, initiating a signaling of this situation.

11. Method of claims 9 or 10, wherein
the subsequent temperature measurements are automatically performed after a lapse of a constant predefined time (Δt).

12. Method of claims 9 or 10, wherein
a time until the subsequent temperature measurement is performed is determined depending on the temperature difference between the previous temperature measurement and the subsequent temperature measurement.

13. Method of anyone of claim 9 to 12, wherein
a first temperature measurement is initiated upon switching on the operating light (1).

14. Method of anyone of claims 9 to 12, wherein
a subsequent temperature measurement is performed as a previous temperature measurement after repositioning of the light head (3).

## Patentansprüche

1. Operationsleuchte (1), aufweisend
einen Lampenkopf (3), der mindestens eine Lichtquelle zum Beleuchten einer Operationsstelle (10) enthält, und
einen Temperatursensor (4), der zum Erfassen einer Temperatur in der Operationsstelle (10) in einer berührungslosen Weise an einer Komponente der Operationsleuchte (1) angebracht ist,
**dadurch gekennzeichnet, dass**
die Operationsleuchte (1) dazu konfiguriert ist, mehrere aufeinanderfolgende Temperaturmessungen durchzuführen und eine Temperaturdifferenz zwischen einer vorausgegangenen Temperaturmessung und einer der nachfolgenden Temperaturmessungen zu bestimmen,
wobei
die Operationsleuchte (1) dazu konfiguriert ist, beim Erreichen oder Überschreiten einer vorbestimmten Differenz zwischen der vorausgegangenen Temperaturmessung und einer der nachfolgenden Temperaturmessungen zumindest eines von einem Verringern einer Lichtintensität der Operationsleuchte (1) und einem Signalisieren durchzuführen.

2. Operationsleuchte (1) nach Anspruch 1, wobei
die Operationsleuchte (1) eine erste mechanische und elektrische Schnittstelle (6) aufweist,
der Temperatursensor (4) eine Komponente einer Temperatursensorbaugruppe (5) ist, und
die Temperatursensorbaugruppe (5) eine zweite mechanische und elektrische Schnittstelle (7) aufweist, die dazu konfiguriert ist, an die erste mechanische und elektrische Schnittstelle (6) gekoppelt zu werden.

3. Operationsleuchte (1) nach Anspruch 2, wobei
die erste mechanische und elektrische Schnittstelle (6) in dem Zentrum des Lampenkopfs (3) auf einer Seite einer Lichtaustrittsfläche (3') des Lampenkopfs (3) angeordnet ist.

4. Operationsleuchte (1) nach Anspruch 2 oder 3, wobei
die erste mechanische und elektrische Schnittstelle (6) dazu konfiguriert ist, eine Kamera an der Operationsleuchte (1) anzubringen und elektrisch zu verbinden.

5. Operationsleuchte (1) nach einem der vorangegangenen Ansprüche, wobei der Temperatursensor (4) ein Infrarottemperatursensor ist.

6. Operationsleuchte (1) nach einem der Ansprüche 1 bis 4, wobei der Temperatursensor (4) eine Wärmebildkamera ist.

7. Operationsleuchte (1) nach einem der vorangegangenen Ansprüche, wobei
die Operationsleuchte (1) dazu konfiguriert ist, nachzuweisen, ob die erfasste Temperatur innerhalb eines vorbestimmten Temperaturbereichs liegt.

8. Operationsleuchte (1) nach einem der vorangegangenen Ansprüche, wobei
die Operationsleuchte (1) mit einem Bewegungssensor (12) versehen ist, und
die Operationsleuchte (1) dazu konfiguriert ist, eine Temperaturmessung nach einer Umpositionierung des Lampenkopfs (3) auszulösen.

9. Verfahren zum Erfassen einer Temperatur in einer Operationsstelle (10) durch eine Operationsleuchte (1) nach einem der Ansprüche 1 bis 8, das die Schritte aufweist:
Durchführen einer vorausgehenden Temperaturmessung und von nachfolgenden Temperaturmessungen;
Bestimmen einer Temperaturdifferenz zwischen einer mit der vorausgehenden Temperaturmessung erfassten Temperatur und einer mit den nachfolgenden Temperaturmessungen erfassten Temperatur; und
Durchführen von zumindest einem von einem von einem Verringern einer Lichtintensität der Operationsleuchte (1) und von einem Signalisieren wenn die bestimmte Temperaturdifferenz eine vorbestimmten Differenz oder eine maximale vorbestimmte Temperatur erreicht oder überschreitet.

10. Verfahren nach Anspruch 9, das die Schritte aufweist:
Nachweisen, ob eine jeweils in der vorausgegangenen Temperaturmessung und in den nachfolgenden Temperaturmessungen erfasste Temperatur in einem vorbestimmten Temperaturbereich liegt; und
wenn die erfasste Temperatur außerhalb des vorbestimmten Temperaturbereichs liegt, Auslösen eines Signalisierens dieser Situation.

11. Verfahren nach Anspruch 9 oder 10, wobei
die nachfolgenden Temperaturmessungen nach einem Ablauf einer konstanten vorbestimmten Zeitdauer (Δt) automatisch durchgeführt werden.

12. Verfahren nach Anspruch 9 oder 10, wobei
eine Zeitdauer, bis die nachfolgende Temperaturmessung durchgeführt wird, in Abhängigkeit von der Temperaturdifferenz zwischen der vorausgehenden Temperaturmessung und der nachfolgenden Temperaturmessung bestimmt wird.

13. Verfahren nach einem der Ansprüche 9 bis 12, wobei
eine erste Temperaturmessung beim Einschalten der Operationsleuchte (1) ausgelöst wird.

14. Verfahren nach einem der Ansprüche 9 bis 12, wobei
eine nachfolgende Temperaturmessung nach einem Umpositionieren des Lampenkopfs (3) als eine vorausgehende Temperaturmessung durchgeführt wird.

## Revendications

1. Scialytique (1) comprenant
une tête lumineuse (3) incluant au moins un illuminant pour illuminer un site chirurgical (10), et
un capteur de température (4) fixé à un constituant de scialytique (1) pour détecter une température dans le site chirurgical (10) dans une manière sans contact,
**caractérisé en ce que**
le scialytique (1) est configuré pour réaliser plusieurs mesures successives de température et pour déterminer une différence de température entre une mesure de température antérieure et une de mesures de température subséquentes,
dans lequel
le scialytique (1) est configuré pour réaliser au moins un d'une diminution d'intensité de lumière de scialytique (1) et d'un signalement d'avoir atteint ou dépassé une différence prédéfinie entre la mesure de température antérieure et une des mesures de température subséquentes.

2. Scialytique (1) selon la revendication 1, dans lequel
le scialytique (1) comprend une première interface mécanique et électrique (6),
le capteur de température (4) est un constituant d'un assemblage de capteur de température (5), et
l'assemblage de capteur de température (5) comprend une seconde interface mécanique et électrique (7) configurée pour être couplée à la première interface mécanique et électrique (6).

3. Scialytique (1) selon la revendication 2, dans lequel
la première interface mécanique et électrique (6) est disposée au centre de la tête lumineuse (3) sur un côté d'une surface d'émission de lumière (3') de la tête lumineuse (3).

4. Scialytique (1) selon la revendication 2 ou 3, dans lequel
la première interface mécanique et électrique (6) est configurée pour fixer et connecter électriquement une caméra au scialytique (1).

5. Scialytique (1) selon l'une quelconque des revendications antérieures, dans lequel
le capteur de température (4) est un capteur de température à infrarouge.

6. Scialytique (1) selon l'une quelconque des revendications 1 à 4, dans lequel
le capteur de température (4) est une caméra thermique.

7. Scialytique (1) selon l'une quelconque des revendications antérieures, dans lequel
le scialytique (1) est configuré pour vérifier si la température détectée se trouve dans un intervalle de température prédéfini.

8. Scialytique (1) selon l'une quelconque des revendications antérieures, dans lequel
le scialytique (1) est muni d'un capteur de déplacement (12), et
le scialytique (1) est configuré pour initier une mesure de température après repositionnement de la tête lumineuse (3).

9. Procédé pour détecter une température dans un site chirurgical (10) par un scialytique (1) selon l'une quelconque des revendications 1 à 8 comprenant les étapes de :
- réalisation d'une mesure de température antérieure et de mesures de température subséquentes ;
- détermination d'une différence de température entre une température détectée par la mesure de température antérieure et une température détectée par les mesures de température subséquentes ; et
- réalisation d'au moins un d'une diminution d'intensité de lumière de scialytique (1) et d'un signalement lorsque la différence de température déterminée atteint ou dépasse une différence de température prédéfinie ou une température prédéfinie maximale.

10. Procédé selon la revendication 9 comprenant les étapes de :
- vérification si une température respectivement détectée dans la mesure de température antérieure et dans les mesures de température subséquentes se trouve dans un intervalle de température prédéfini ; et
- lorsque la température détectée est à l'extérieur de l'intervalle de température prédéfini, initiation d'une signalisation de cette situation.

11. Procédé selon la revendication 9 ou 10, dans lequel
les mesures de température subséquentes sont automatiquement réalisées après un laps de temps prédéfini constant (ΔT).

12. Procédé selon la revendication 9 ou 10, dans lequel
une durée jusqu'à ce que la mesure de température subséquente est réalisée est déterminée selon la différence de température entre la mesure de température antérieure et la mesure de température subséquente.

13. Procédé selon l'une quelconque des revendications 9 à 12, dans lequel
une première mesure de température est initiée par commutation sur scialytique (1).

14. Procédé selon l'une quelconque des revendications 9 à 12, dans lequel
une mesure de température subséquente est réalisée comme une mesure de température antérieure après repositionnement de la tête lumineuse (3).
